# EUROPÄISCHE PATENTANMELDUNG

(11) **EP 0 633 034 A1**
(43) Veröffentlichungstag der Anmeldung: **11.01.1995**
(21) Anmeldenummer: 94108409.7
(22) Anmeldetag: 01.06.1994
(51) Int. Cl.: A61M 5/162, A61M 39/20

(54) **Infusionsbesteck**

(30) Priorität: 12.06.1993 DE 4319510
(71) Anmelder: B. BRAUN MELSUNGEN AG, D-34212 Melsungen (DE)
(72) Erfinder: Lesemann, Egon, D-34212 Melsungen (DE); Fuchs, Jürgen, D-34308 Bad Emstal (DE)
(74) Vertreter: Selting, Günther, Dipl.-Ing.

(57) **Zusammenfassung**

Das medizinische Gerät ist an seinen Enden mit jeweils einem Siegel (18;30) versehen, das beim erstmaligen Öffnen zerreißt und dadurch eine Originalitätssicherung bildet. Das Siegel (18) ist vorzugsweise keimdicht, so daß die Innensterilität des Gerätes gewahrt bleibt. Dadurch kann auf eine keimdichte Außenverpackung in bestimmten Fällen verzichtet werden.

## Beschreibung

Die Erfindung betrifft ein medizinisches Gerät und insbesondere ein Infusions- oder Transfusionsgerät, das an den Enden Öffnungen zum verbinden des Geräteinneren mit einem externen Gerät aufweist.

Medizinische Geräte, die zum einmaligen Gebrauch hergestellt werden, wie z.B. Infusions- oder Transfusionsgeräte, Verbindungsleitungen, Kanülen, Spritzen u.dgl., sind an den Anschlußstellen, an denen die Verbindung zu einem anderen medizinischen Gerät erfolgt, häufig mit Luer-Lok-Verbindungen versehen, deren Öffnungen unverschlossen sind. An Stellen, an denen Verletzungsgefahr (z.B. durch Dorne oder Kanülen) für den Anwender besteht oder die Gefahr der Beschädigung der Sterilverpackung droht, sind die medizinischen Geräte mit einer Schutzkappe aus Kunststoff ausgerüstet. Die wesentliche Funktion von Schutzkappen sind der Schutz des Anwenders vor Verletzungsgefahr, die Verhinderung der Beschädigung der Sterilverpackung sowie die Vermeidung der Kontamination und/oder Beschädigung der Kontaktierungsstellen.

Ein medizinisches Gerät, von dem die Oberbegriffe der Patentansprüche 1 und 2 ausgehen, ist bekannt aus DE 35 43 124 A1. Dieses medizinische Gerät weist einen Einstechdorn auf, der durch eine abziehbare Schutzkappe bedeckt ist.

Aus DE 31 06 991 A1 ist ein Verschluß für eine medizinische Flasche bekannt, der aus einem den Flaschenverschluß überdeckenden Dichtungsdeckel besteht, welcher an dem Rand der Flaschenöffnung mit einem Klebstoffring befestigt ist. Beim Abziehen des Dichtungsdeckels wird dieser zerstört, wobei sein Rand an der Flaschenöffnung hängenbleibt. Dies kennzeichnet das erstmalige Öffnen der Flasche.

Aus DE-OS 27 53 239 ist eine Flasche bekannt, bei der über den Grenzbereich zwischen Flaschenkörper und abnehmbarem Verschluß ein Streifen geklebt ist, der bei Entfernen des Verschlusses reißt.

In der (nicht vorveröffentlichten) älteren Patentanmeldung P 42 37 436.7 ist eine Schutzkappe für ein medizinisches Gerät beschrieben, bei der der Kappenkörper der Schutzkappe über mindestens ein Abreißteil mit einem nicht zerstörungsfrei lösbaren, an dem Gerät angebrachten Halteteil verbunden ist. Die Schutzkappe besteht hierbei aus dem Kappenkörper und dem Halteteil, das dem Kappenkörper angeformt ist. Beim Abnehmen des Kappenkörpers reißt das Abreißteil, so daß der Kappenkörper von dem Halteteil abgetrennt wird, das am Gerät verbleibt und damit anzeigt, daß eine Dekonnektierung des Kappenkörpers erfolgt ist. Hierdurch wird eine Originalitätsgarantie bewirkt, indem das erstmalige Dekonnektieren des Kappenkörpers erkennbar ist. Wird beim Abnehmen des Kappenkörpers festgestellt, daß das Abreißteil bereits zerbrochen ist, dann wird erkannt, daß das Gerät nicht mehr im originalitätszustand ist.

Der Erfindung liegt die Aufgabe zugrunde, ein medizinischas Gerät zu schaffen, bei dem eine Originalitätssicherung auf einfache Weise kostengünstig bereitgestellt wird.

Die Lösung dieser Aufgabe erfolgt erfindungsgemäß mit den Merkmalen des Patentanspruchs 1.

Nach der Erfindung ist die auf das Gerät aufgesetzte Schutzkappe durch ein zerreißbares Siegel mit dem Gerät verbunden. Beim erstmaligen Abziehen der Schutzkappe zerbricht das Siegel und zeigt dadurch dem Benutzer an, daß das Gerät nicht mehr im Originalzustand ist. Die Innensterilität des Gerätes ist garantiert, solange das Siegel nicht zerbrochen ist. Daher kann auf eine keimdichte Verpackung des Gerätes verzichtet werden.

Im folgenden werden unter Bezugnahme auf die Zeichnungen Ausführungsbeispiele der Erfindung näher läutert.

Es zeigen:
- Fig. 1: eine schematische Darstellung einer ersten Ausführungsform eines medizinischen Gerätes,
- Fig. 2: einen Längsschnitt durch die Einzelheit II aus Fig. 1,
- Fig. 3: eine Draufsicht auf Fig. 2,
- Fig. 4: einen Längsschnitt durch die Einzelheit IV von Fig. 1 und
- Fig. 5: eine Ansicht des einen Siegels.

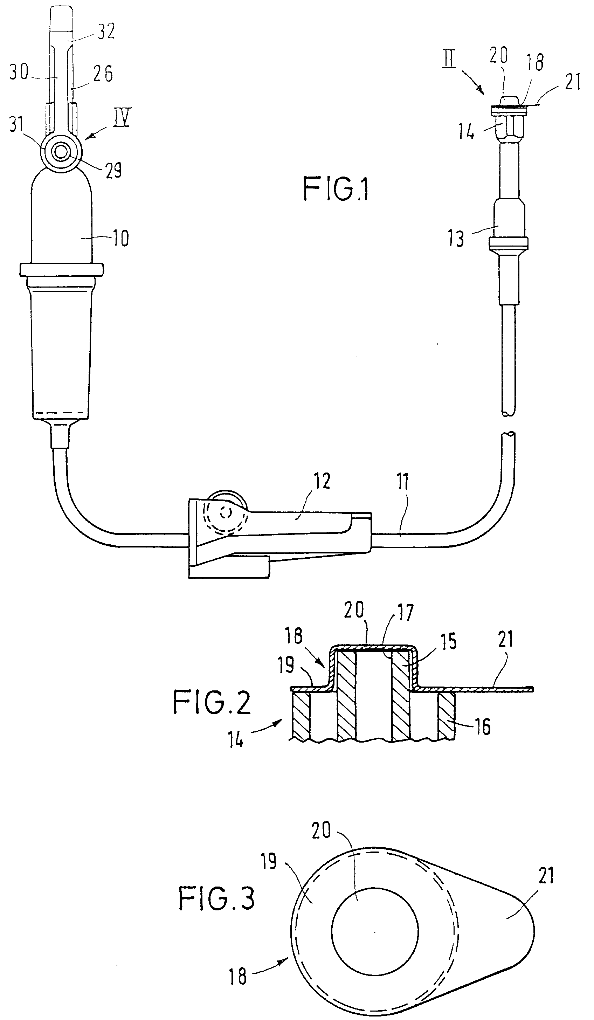

Das in Fig. 1 dargestellte medizinische Gerät besteht aus einer Tropfkammer 10, einem daran angeschlossenen Schlauch 11, auf dem eine Rollenklemme 12 sitzt, einem Injektionszwischenstück 13 und einem Anschlußstück 14.

Gemäß Fig. 2 weist das Anschlußstück 14 ein Rohr 15 auf, das in axialer Richtung über einen umgebenden Mantel 16 übersteht. Das Ende des Rohres 15 bildet die Öffnung 17, die mit dem Innern des Schlauchs 11 verbunden ist.

Das stirnseitige Ende des Anschlußstücks 14 ist mit einem Siegel 18 verschlossen, das aus Blattmaterial besteht, beispielsweise aus Papier oder Folie aus Metall oder Kunststoff. Das Siegel 18 ist durch Tiefziehen oder durch faltenbildendes Pressen so verformt, daß es einen flanschartig umlaufenden Rand 19 bildet, der eine Vertiefung 20 umgibt. Die Vertiefung 20 ist passend auf das Ende des Rohres 15 aufgesetzt und der Rand 19 ist an dem stirnseitigen Ende des Mantels 16 verklebt oder verschweißt, so daß ein dichter Verschluß des Mantels 16 entsteht. Von dem Rand 19 steht nach einer Seite eine Grifflasche 21 ab.

Auf die beschriebene Weise ist das eine Ende des medizinischen Gerätes keimdicht verschlossen. Zur Benutzung des Gerätes wird das Siegel 18 durch Angreifen an der Grifflasche 21 abgezogen, wobei es sich von dem Anschlußstück 14 löst. Anschließend kann das Siegel 18 nicht wieder an dem Anschlußstück 14 befestigt werden, weil es dann nicht mehr haftet.

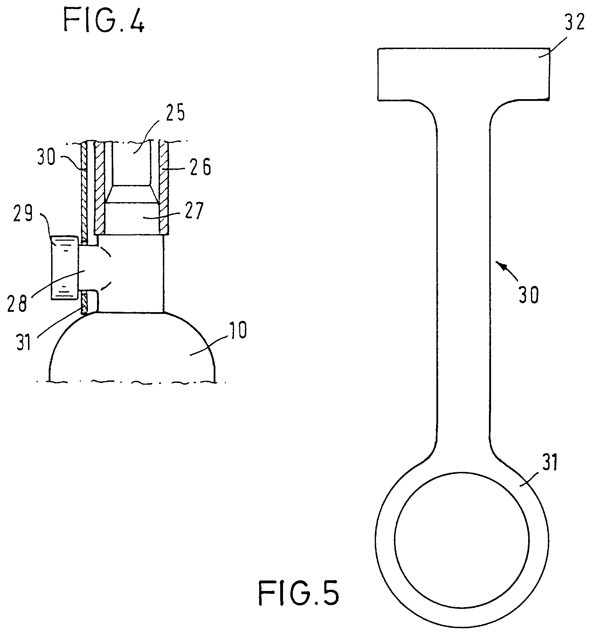

An dem anderen Ende des gegen die Umgebung verschlossenen-medizinischen Gerätes befindet sich an der Tropfkammer 10 ein Einstechdorn 25 (Fig. 4), auf dem eine Schutzkappe 26 sitzt. Der Einstechdorn hat an seinem in Fig. 4 nicht sichtbaren vorderen Ende Öffnungen, die als Flüssigkeitseinlaß und Belüftungsöffnung dienen und in das Innere der Tropfkammer 10 hineinführen. Die Schutzkappe 26 umgibt den Einstechdorn 25 vollständig und ihr unteres Ende sitzt abdichtend auf einem Sitz 27 des Einstechdornes. Zur Benutzung des Gerätes wird die Schutzkappe 26 abgezogen, so daß der Einstechdorn 25 freiliegt, um durch den Elastomerstopfen einer Flasche hindurchgestochen zu werden.

Von einem Rohrstück, das die Tropfkammer 10 mit dem Einstechdorn 25 verbindet, steht ein seitlicher Rohrstutzen 28 ab, der ein Belüftungsfilter enthält, das von einer von außen aufgesetzten Kappe 29 festgehalten wird. Bevor die Kappe 29 aufgesetzt wurde, ist das in Fig. 5 dargestellte Siegel 30 angebracht worden. Dieses Siegel besteht aus einem Blattmaterialstreifen, der an einem Ende einen geschlossenen Ring 31 und am entgegengesetzten Ende einen Befestigungsstreifen 32 aufweist. Der Ring 31 ist auf den Rohrstutzen 28 aufgesetzt und anschließend ist die Kappe 29 befestigt worden, so daß das Siegel 30 unverlierbar an dem Gerät befestigt ist. Dann wird der Befestigungsstreifen 32 an die Schutzkappe 26 angeklebt.

Um den Einstechdorn 25 freizulegen, ist es erforderlich, die Schutzkappe 26 abzuziehen. Dabei reißt unvermeidlich das Siegel 30 auf. Der Ring 31 bildet den schwächsten Teil des Siegels 30, so daß das Material am Ring 31 an zwei Stellen aufreißt. Dies ist nach außen hin-sichtbar und zeigt an, daß das Gerät nicht mehr im Originalitätszustand ist.

Die Schutzkappe 26 bildet mit dem Sitz 27 einen keimdichten Verschluß. Die Dichtigkeit dieses Verschlusses ist so lange garantiert wie das Siegel 30 unbeschädigt ist.

## Patentansprüche

1. Medizinisches Gerät mit mindestens einem eine Öffnung (17) aufweisenden Anschlußstück (14) zum Verbinden des Geräteinnern mit einem externen Gerät,
**dadurch gekennzeichnet,**
daß an dem Anschlußstück (14) ein die Öffnung (17) verschließendes, abziehbares Siegel (18) angebracht ist, das nach dem Abziehen nicht wieder an dem Anschlußstück (14) haftet.

2. Medizinisches Gerät mit mindestens einem eine Öffnung aufweisenden Anschlußstück (Einstechdorn 25) zum verbinden des Geräteinnern mit einem externen Gerät,
**dadurch gekennzeichnet,**
daß an einer das Anschlußstück (25) bedeckenden abnehmbaren Schutzkappe (26) und an dem Gerät ein aus Blattmaterial bestehendes Siegel (30) befestigt ist, das beim Abziehen der Schutzkappe (26) zerstört wird.

3. Medizinisches Gerät nach Anspruch 1 oder 2, dadurch gekennzeichnet, daß das Siegel (18;30) keimdicht ist.

4. Medizinisches Gerät nach Anspruch 2, dadurch gekennzeichnet, daß das Siegel (30) an der Schutzkappe (26) angeklebt ist und einen Ring (31) aufweist, der einen Ansatz (28) des Gerätes umgibt.

5. Medizinisches Gerät nach einem der Ansprüche 1-4, dadurch gekennzeichnet, daß das Gerät ein Infusions- oder Transfusionsgerät ist, dessen beide Enden mit einem Siegel (18) oder mit einer durch ein Siegel (30) gesicherten Schutzkappe (26) versehen sind.

6. Medizinisches Gerät nach einem der Ansprüche 1-5, dadurch gekennzeichnet, daß das Siegel (18,30) eine Sollbruchlinie (52,54) aufweist.
